(19) Europäisches Patentamt
European Patent Office
Office européen des brevets

(11) EP 2 890 362 B1

(12) **EUROPEAN PATENT SPECIFICATION**

(45) Date of publication and mention of the grant of the patent:
**11.11.2020 Bulletin 2020/46**

(21) Application number: **13759935.3**

(22) Date of filing: **27.08.2013**

(51) Int Cl.:
***A61K 8/37*** (2006.01) ***A61K 8/43*** (2006.01)
***A61Q 19/00*** (2006.01) ***A61K 9/08*** (2006.01)
***A61K 47/14*** (2017.01) ***A61K 31/155*** (2006.01)
***A61P 31/04*** (2006.01)

(86) International application number:
**PCT/US2013/056802**

(87) International publication number:
**WO 2014/035971 (06.03.2014 Gazette 2014/10)**

(54) **CHLORHEXIDINE GLUCONATE SOLUBILIZED IN A HYDROPHOBIC MONOACYLGLYCERIDE**

IN EINEM HYDROPHOBEN MONOACYLGLYCERID SOLUBILISIERTES CHLORHEXIDINGLUCONAT

GLUCONATE DE CHLORHEXIDINE SOLUBILISÉ DANS DU MONOACYLGLYCÉRIDE HYDROPHOBE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.08.2012 US 201261694080 P**

(43) Date of publication of application:
**08.07.2015 Bulletin 2015/28**

(73) Proprietor: **3M Innovative Properties Company**
**St. Paul, MN 55133-3427 (US)**

(72) Inventors:
- **MENON, Vinod P.**
**Saint Paul, Minnesota 55133-3427 (US)**
- **RULE, Joseph D.**
**Saint Paul, Minnesota 55133-3427 (US)**
- **ROSS, Richard B.**
**Saint Paul, Minnesota 55133-3427 (US)**

(74) Representative: **Vossius & Partner**
**Patentanwälte Rechtsanwälte mbB**
**Siebertstrasse 3**
**81675 München (DE)**

(56) References cited:
**FR-A1- 2 379 284** **JP-A- H0 399 010**
**JP-A- 2007 217 394** **US-A- 5 017 617**
**US-A1- 2008 064 711** **US-A1- 2010 022 654**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD

The present disclosure relates to chlorhexidine gluconate solubilized in a hydrophobic monoacylglyceride. The present disclosure also relates to compositions incorporating such material, as well as methods of preparing such materials.

### SUMMARY

Briefly, in one aspect, the present disclosure provides a composition comprising chlorhexidine gluconate solubilized in a monoacylglyceride having a hydrophilic-lipophilic balance of no greater than 10 according to claim 1.

In some embodiments, the monoacylglyceride has a solubility parameter of greater than 10 $(cal/cm^3)^{1/2}$ (42 $(J/cm^3)^{1/2}$). In some embodiments, the monoacylglyceride has a binding energy to chlorhexidine gluconate of at least 25 kilocalories per mole (105 kilojoules per mole). In some embodiments, the monoacylglyceride comprises vicinal hydrogen-bonding groups.

The acyl group of the monoacylglyceride is a C8 to C18 acyl group, e.g., glycerol monocaprylate, glycerol monolaurate, glycerol monoisostearate, glycerol monooleate, and combinations thereof.

In some embodiments, the composition comprises no greater than 1 part by weight monoacylglyceride per 1 part by weight chlorhexidine gluconate. In some embodiments, the composition comprises no greater than 0.1 parts by weight water per 1 part by weight chlorhexidine gluconate.

In another aspect, the present disclosure provides the composition further comprises a carrier. In some embodiments, the carrier comprises one or more of a hydrophobic vehicle other than a monoacylglyceride, a polymer, and a solvent. In some embodiments, the composition further comprises a thixotropic agent.

The above summary of the present disclosure is not intended to describe each embodiment of the present invention. The details of one or more embodiments of the invention are also set forth in the description below.

### DETAILED DESCRIPTION

Chlorhexidine digluconate, commonly referred to as "chlorhexidine gluconate" or "CHG," is an antimicrobial useful in various applications. CHG is often provided as an aqueous solution, in part because CHG may degrade in a non-aqueous composition. CHG has been provided in non-aqueous solutions by replacing water with a hydrophilic vehicle. For example, U.S. Patent No. 6,458,341 (Rozzi et al., issued October 1, 2002) describes non-aqueous solutions containing CHG and a solubilizing glycol.

Oil based compositions comprising CHG dispersed in mono esters of sorbitol are described in FR 2 379 284 A1.

Surprisingly, the present inventors have discovered that CHG can be solubilized in a hydrophobic vehicle. Consistent with typical usage, as used herein, a "hydrophobic vehicle" is one having a hydrophile/lipophile balance ("HLB") of no greater than 10. For example, in some embodiments, the compositions comprise at least 5% by weight CHG dissolved in the hydrophobic vehicle based on the combined weight of the CHG and hydrophobic vehicle. In some embodiments, the compositions comprise at least 10%, e.g., at least 15%, or even at least 20% by weight CHG dissolved in the hydrophobic vehicle based on the combined weight of the CHG and hydrophobic vehicle.

Three distinct methods are available for preparing non-aqueous solutions of CHG in hydrophobic vehicles. The first method involves lyophilizing CHG, and then dissolving the CHG into the hydrophobic vehicle. The second method involves mixing an aqueous CHG solution with a relatively high boiling hydrophobic vehicle, and then pulling a vacuum on the mixture to remove the water. The third method involves generating the CHG *in situ* by reacting gluconolactone, a limited amount of water, and chlorhexidine free base. Each method is expected to give a similar final product, i.e., CHG dissolved in a hydrophobic vehicle, but each method has advantages and disadvantages. For example, the lyophilization route does not require exposing the CHG to sustained heat, which helps prevent degradation. The liquid vacuum stripping route is easily scalable using readily available manufacturing equipment, e.g., kettles. The *in situ* generation method does not require vacuum-equipped reactors. All the methods may leave small amounts of water behind. Therefore, as used herein, "non-aqueous" refers to compositions that may contain small amounts of water, i.e. less than 5 wt.%, based on the total weight of the composition. In some embodiments, the compositions contain less than 2 wt.%, e.g., less than 1 wt.%, or even less than 0.1 wt.% water, based on the total weight of the composition.

The compositions contain little or no hydrophilic vehicle, i.e., vehicles having an HLB of greater than 10. As used herein, water is considered a separate component independent of any hydrophilic vehicles; therefore, the following amounts are exclusive of any water which may be present in the composition. The compositions comprise no greater than 1 part by weight hydrophilic vehicle per 1 part by weight CHG, e.g., no greater than 0.5 part by weight, or even no greater than 0.1 parts by weight hydrophilic vehicle per 1 part by weight CHG.

In some embodiments, the compositions comprise no greater than 1 part by weight water per 1 part by weight CHG, e.g., no greater than 0.5 part by weight, no greater than 0.1 part by weight, or even no greater than 0.01 parts by weight water per 1 part by weight CHG.

Hydrophobic vehicles have an HLB value of no greater than 10. In some embodiments, the hydrophobic vehicle has an HLB value of no greater than 9, e.g., no greater than 7.

Generally, the hydrophobic vehicle is a monoacylglyceride, e.g., 1-monoacylglycerides having the formula:

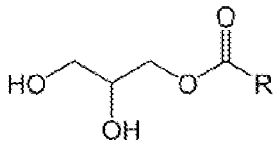
(I);

and 2-monoacylglycerides having the formula:

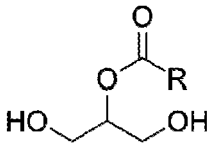
(II);

wherein RC=O is the acyl group. Such monoacylglycerides include proximate hydroxyl groups. As used herein, "proximate" groups refer to groups separated by no more than three carbon atoms, e.g., the 2-monoacylglycerides of Formula II. In some embodiments, the proximate groups may be vicinal, i.e., separated by two carbon atoms, e.g., the 1-monoacylglycerides of Formula I.

The acyl group of the monoacylglyceride is a C8 to C18 acyl group. In some embodiments, the R-group of the acyl group is linear. In some embodiments, the R-group is branched. In some embodiments, the R-group is saturated. In some embodiments, the R-group is unsaturated. Often monoacylglycerides are available as a mixture containing a range of acyl group chain lengths. Exemplary monoacylglycerides include, e.g., glycerol monocaprylate, glycerol monolaurate, glycerol monoisostearate, glycerol monooleate, and combinations thereof. Often such materials are commercially available as mixtures containing both the 1-monoacylglyceride and the 2-monoacylglyceride.

Examples. Objects and advantages of various embodiments of the present disclosure are further illustrated by the following examples, but the particular materials and amounts thereof recited in these examples, as well as other conditions and details, should not be construed to unduly limit this invention. Unless otherwise indicated, all parts and percentages are on a weight basis, all water is distilled water, and all molecular weights are weight average molecular weight.

General Method for Vacuum-Stripping Water from CHG Solutions. A 25 g sample of CHG/water solution (20 wt. CHG) was added to 45 g of the hydrophobic monoacylglyceride of interest in a 200 mL round bottom flask. The flask was put in a 60 °C oil bath and stirred with a magnetic stir bar. Vacuum (less than 3.3 kilopascal (25 Torr)) was pulled until bubbling stopped and the weight had closely approached the theoretical value of 50 grams - usually 30-90 minutes. In some cases, the hydrophobic monoacylglyceride was somewhat volatile and the weight of the solution decreased significantly below 50 grams. In those cases, after nearly all the water was removed, additional hydrophobic monoacylglyceride was added to bring the final weight to 50 grams.

Screening tests were conducted to determine the solubility of CHG in a variety of hydrophobic vehicles. Samples were prepared by mixing CHG (20 wt.% solution in water, obtained from Xttrium Laboratories Inc., Chicago, Illinois) with the vehicle and vacuum stripping in accordance with the General Method for Vacuum-Stripping Water from CHG Solutions. The resulting concentration of CHG and residual water were determined. Also, the final state of the solution was qualitatively evaluated to determine whether the mixture was transparent and apparently homogenous, or clearly inhomogeneous. The results obtained using compounds with ester groups are summarized in Tables 1 and 2.

Table 1: Vehicles with ester groups providing good CHG solubility.

| Compound | Alkyl group | CHG. wt.% | Water wt.% | Temperature | Final State |
|---|---|---|---|---|---|
| Glyceryl monocaprylate | C8 | 20% | ND | 23 - 60 °C | Homogeneous |
| Glyceryl monolaurate | C12 | 20% | 0.7% | 75 °C | Homogeneous |
| Glyceryl monooleate | C18 | 10% | 0.3% | 23 - 60 °C | Homogeneous |

(continued)

| Compound | Alkyl group | CHG. wt.% | Water wt.% | Temperature | Final State |
|---|---|---|---|---|---|
| Glyceryl monostearate | C18 | 10% | 0.2% | 80 °C | Homogeneous |
| Glyceryl monoisostearate | C18 | 20% | ND | 60 °C | Homogeneous |
| | | 16% | 0.1% | 23 - 60 °C | Homogeneous |
| | | 10% | 0.7% | 23 - 60 °C | Homogeneous |
| Medium Chain Monoglyceride | mix | 20% | 0.3% | 23 - 50 °C | Homogeneous |
| ND = not determined | | | | | |

Table 2: Vehicles with ester groups providing poor CHG solubility.

| Compound | CHG wt.% | Temperature | Final State |
|---|---|---|---|
| Dialkyl (C12 & C13) Tartrate | 10% | 23 - 50 °C | Inhomogeneous |
| Trigyceryl diisostearate | 10% | 23 - 60 °C | Inhomogeneous |
| | 1% | 23 - 60 °C | Inhomogeneous |
| Polyglycerol-2-Triisostearate | 10% | 23 - 60 °C | Inhomogeneous |

Molecular dynamics simulations were used to investigate underlying causes of observed differences in solubilities with CHG. Simulations were independently carried out to find optimal binding structures of several of the hydrophobic vehicles with CHG. Solubility parameters were also been computed via molecular dynamics simulations for several of the vehicles.

HLB values were calculated using the method of Griffin (Griffin WC; J. Soc. of Cosmetic Chemists 5, 259 (1954)) (the "HLB Method"). In this method,

$$HLB = (E + P) / 5,$$

where E is the weight percent of oxyethylene content and P is the weight percent of polyhydric alcohol content (glycerol, sorbitol, etc). For the compounds herein, glycerol segments with two hydroxyl groups, glycerol segments with one hydroxyl group, and hydroxyl-containing segments of any additional polyhydric molecules were included in the definition of P.

Other methods of calculating HLB are available and may be required when determining the HLB value for compounds lacking both E and P groups, as defined above. While the calculated value of HLB may vary depending on the method used, the trends and relative hydrophobicity of materials are expected to be similar.

The Solubility Parameter Method. Solubility parameters were computed via molecular dynamics simulations using the general procedures as described by Belmares et al. (Belmares, M.; Blanco, M.; Goddard, W.A.; Ross, R.B.; Caldwell, G; Chou, S.H.; Pham, J.; Olofson, P.M.; Thomas, C.; J. Comp. Chem., 25 (15), 1814 (2004), as implemented in Culgi software (Culgi Software, Culgi BV, P.O. Box 252, 2300 AG Leiden, The Netherlands).

The Binding Energy Method. Binding energies were computed using quench molecular dynamics simulations employing QEq charges (Rappe, A.K.; Goddard, W.A.; J. Phys. Chem. 95, 3358 (1991)) and the Dreiding Force Field (Mayo, S.L., Olafson, B.D., Goddard, W.A.; J. Phys. Chem. 94, 8897 (1990)) as implemented in Materials Studio Software (Materials Studio, Accelrys, Software Inc., 10188 Telesis Court, Suite 100, San Diego, CA 92121). Multiple independent simulations were carried out for each vehicle / CHG pair. Typically, 100,000 steps of simulation at 298K were carried out between the hydrophobic vehicle of interest and CHG. The simulation was typically "quenched" or minimized into an optimized complex structure every 5000 steps. Based on the results of independent simulations, the binding energy for the most optimized (lowest energy) complex structure was found using the equation:

$$\text{Binding Energy} = \frac{E(HV) + E(CHG) - E(HV*CHG)}{HBN}$$

wherein E(HV) is the energy of the hydrophobic vehicle, E(CHG) is the energy of the CHG, and E(CHG*HV) is the energy of the CHG*hydrophobic vehicle complex. The Hydrogen Bonding Number (HBN) is a normalization factor to account for varied numbers of hydrogen bonding groups in the hydrophobic vehicles. HBN was computed as a weighted sum of the number of hydrogen-bonding donor/acceptor groups and hydrogen-bonding acceptor-only groups. The hydrogen-bonding donor/acceptor groups (e.g., OH) were given a weighting of 1. The hydrogen-bonding acceptor-only groups (e.g., O in C=O or O in C-O-C) were given a weighting of 1/2. This general procedure was similar to that used by Zhang et al. to compute binding energies between polymers and hydroxy apatite (Zhang, H.P.; Xiong, L; Leng; Y, Fang, L.; Qu, Shuxin; Feng, B.; Weng, J.; Wang, J.; Acta Biomaterialia 5(4), 1169 (2009)). In the present application, the binding energies were computed between organic compounds as opposed to between an organic and inorganic compound as in the Zhang et al. studies.

A summary of calculated HLB, solubility parameter, and binding energy for various hydrophobic vehicles with CHG are shown in Table 3, along with CHG experimental solubility data for comparison. As can be seen in this table, vehicles with a computed solubility parameter greater than 10 were experimentally found to provide CHG-solubility, while those with a computed solubility parameter of less than 10 did not. In addition, vehicles that were experimentally found to provide CHG-solubility had computed CHG binding energies of greater than 25 kcal/mol (105 kJ/mol).

Table 3: Calculated Solubility Parameter, Normalized Binding Energy, and CHG-solubility for a variety of hydrophobic vehicles. 1 cal corresponds to 4.2 J.

| Hydrophobic Vehicle | HLB | Solubility Parameter $(cal/cm^3)^{1/2}$ | Binding Energy (kcal/mol) | Experimental Solubility with CHG |
|---|---|---|---|---|
| Glyceryl monocaprylate | 8.4 | 13.1 | 27 | Yes |
| Glyceryl monolaurate | 6.6 | 13.9 | 29 | Yes |
| Glycerol monooleate | 5.1 | 10.8 | 28 | Yes |
| Glyceryl monostearate | 5.1 | 10.8 | 32 | Yes |
| Glyceryl monoisostearate | 5.1 | 10.8 | 28 | Yes |
| Dialkyl (C12 & C13) Tartrate | 4.2 | 9.6 | 21 | No |
| Trigyceryl diisostearate | 5.8 | 9.6 | 14 | No |
| Polyglycerol-2-Triisostearate | 3.4 | 8.5 | 32 | No |

Analysis of optimized molecular simulation binding structures indicated that sterically unhindered vehicles with proximate hydrogen bonding groups can form exceptionally high binding complexes with CHG due to multiple hydrogen bonds between CHG and the vehicle. The proximate hydrogen bonding groups in the vehicle act in a synergistic binding manner to enable the highly hydrogen-bound complexes much like the mechanical action of a zipper in which zipping one-link enables easier zipping of the next link. Separating or adding steric hindrance to regions near the proximate hydrogen bonding groups can prevent this synergy.

In some embodiments, the compositions may include a carrier in addition to the monoacylglyceride vehicle. In some embodiments, the carrier may comprise another hydrophobic vehicle. In some embodiments, the carrier may comprise a polymer. In some embodiments, the carrier may comprise a solvent. In some embodiments, the carrier may comprise a thixotropic agent, i.e., an agent which renders the composition shear thinning.

## Claims

1. A composition comprising chlorhexidine digluconate solubilized in a monoacylglyceride, wherein the monoacylglyceride is hydrophobic monoacylglyceride having a hydrophilic-lipophilic balance of no greater than 10 as determined using the HLB Method;
   wherein the composition comprises at least 5% by weight chlorhexidine digluconate dissolved in the hydrophobic monoacylglyceride based on the combined weight of the CHG and hydrophobic monoacylglyceride;
   wherein the acyl group of the monoacylglyceride is a C8 to C18 acyl group;
   wherein the composition comprises less than 5 wt.% water based on the total weight of the composition; and

wherein the composition comprises no greater than 1 part by weight hydrophilic vehicle per 1 part by weight chlorhexidine digluconate, wherein water is considered a separate component independent of any hydrophilic vehicles.

2. The composition of claim 1, wherein the monoacylglyceride has a solubility parameter of greater than 10 $(cal/cm^3)^{1/2}$ (42 $(J/cm^3)^{1/2}$).

3. The composition according to any one of the preceding claims, wherein the monoacylglyceride has a binding energy to chlorhexidine digluconate of at least 25 kilocalories per mole (105 kilojoules per mole).

4. The composition according to any one of the preceding claims, wherein the monoacylglyceride comprises two vicinal hydrogen-bonding groups.

5. The composition of any one of the preceding claims, wherein the monoacylglyceride is selected from the group consisting of glycerol monocaprylate, glycerol monolaurate, glycerol monoisostearate, glycerol monostearate, glycerol monooleate, and combinations thereof.

6. The composition of any one of the preceding claims, wherein the monoacylglyceride is selected from the group consisting of glycerol monocaprylate, glycerol monolaurate, glycerol monoisostearate, glycerol monooleate, and combinations thereof.

7. The composition according to any one of the preceding claims, wherein the composition comprises no greater than 0.1 parts by weight water per 1 part by weight chlorhexidine digluconate.

8. The composition according to any one of the preceding claims, further comprising a carrier.

9. The composition of claim 8, wherein the carrier comprises a polymer.

10. The composition of claim 8 or 9, wherein the carrier comprises an organic solvent.

11. The composition according to any one of the preceding claims, further comprising a thixotropic agent.

**Patentansprüche**

1. Eine Zusammensetzung, umfassend Chlorhexidindigluconat, gelöst in einem Monoacylglycerid, wobei das Monoacylglycerid ein hydrophobes Monoacylglycerid mit einem hydrophil-lipophilen Anteil von nicht mehr als 10 ist, wie unter Verwendung des HLB-Verfahrens bestimmt;
wobei die Zusammensetzung mindestens 5 Gew.-% Chlorhexidindigluconat umfasst, gelöst in dem hydrophoben Monoacylglycerid, bezogen auf das kombinierte Gewicht des CHG und des hydrophoben Monoacylglycerids;
wobei die Acylgruppe des Monoacylglycerids eine C8- bis C18-Acylgruppe ist;
wobei die Zusammensetzung weniger als 5 Gew.-% Wasser, bezogen auf das Gesamtgewicht der Zusammensetzung, umfasst; und
wobei die Zusammensetzung nicht mehr als 1 Gewichtsteil hydrophiles Vehikel pro 1 Gewichtsteil Chlorhexidindigluconat umfasst, wobei Wasser als separate Komponente betrachtet wird, die von jeglichen hydrophilen Vehikeln unabhängig ist.

2. Die Zusammensetzung nach Anspruch 1, wobei das Monoacylglycerid einen Löslichkeitsparameter von mehr als 10 $(cal/cm^3)^{1/2}$ (42$(J/cm^3)^{1/2}$) aufweist.

3. Die Zusammensetzung nach einem der vorstehenden Ansprüche, wobei das Monoacylglycerid eine Bindungsenergie an Chlorhexidindigluconat von mindestens 25 Kilokalorien pro Mol (105 Kilojoule pro Mol) aufweist.

4. Die Zusammensetzung nach einem der vorstehenden Ansprüche, wobei das Monoacylglycerid zwei vicinale Wasserstoffbindungsgruppen umfasst.

5. Die Zusammensetzung nach einem der vorstehenden Ansprüche, wobei das Monoacylglycerid ausgewählt ist aus der Gruppe, bestehend aus Glycerinmonocaprylat, Glycerinmonolaurat, Glycerinmonoisostearat, Glycerinmonostearat, Glycerinmonooleat und Kombinationen davon.

6. Die Zusammensetzung nach einem der vorstehenden Ansprüche, wobei das Monoacylglycerid ausgewählt ist aus der Gruppe, bestehend aus Glycerinmonocaprylat, Glycerinmonolaurat, Glycerinmonoisostearat, Glycerinmonooleat und Kombinationen davon.

7. Die Zusammensetzung nach einem der vorstehenden Ansprüche, wobei die Zusammensetzung nicht mehr als 0,1 Gewichtsteile Wasser pro 1 Gewichtsteil Chlorhexidingluconat umfasst.

8. Die Zusammensetzung nach einem der vorstehenden Ansprüche, ferner umfassend einen Träger.

9. Die Zusammensetzung nach Anspruch 8, wobei der Träger ein Polymer umfasst.

10. Die Zusammensetzung nach Anspruch 8 oder 9, wobei der Träger ein organisches Lösungsmittel umfasst.

11. Die Zusammensetzung nach einem der vorstehenden Ansprüche, ferner umfassend ein Thixotropikum.

**Revendications**

1. Composition comprenant du digluconate de chlorhexidine solubilisé dans un monoacylglycéride, dans laquelle le monoacylglycéride est un monoacylglycéride hydrophobe ayant un rapport hydrophile-lipophile n'excédant pas 10 tel que déterminé en utilisant le procédé HLB ;
dans laquelle la composition comprend au moins 5 % en poids de digluconate de chlorhexidine dissous dans le monoacylglycéride hydrophobe sur la base du poids combiné du CHG et du monoacylglycéride hydrophobe ;
dans laquelle le groupe acyle du monoacylglycéride est un groupe acyle en C8 à C18;
dans laquelle la composition comprend moins de 5 % en poids d'eau sur la base du poids total de la composition ; et dans laquelle la composition ne comprend pas plus de 1 partie en poids de véhicule hydrophile pour 1 partie en poids de digluconate de chlorhexidine, dans laquelle l'eau est considérée comme étant un composant séparé indépendant de n'importe quels véhicules hydrophiles.

2. Composition selon la revendication 1, dans laquelle le monoacylglycéride a un paramètre de solubilité supérieur à 10 $(cal/cm^3)^{1/2}$ $(42(J/cm^3)^{1/2})$.

3. Composition selon l'une quelconque des revendications précédentes, dans laquelle le monoacylglycéride a une énergie de liaison au digluconate de chlorhexidine d'au moins 25 kilocalories par mole (105 kilojoules par mole).

4. Composition selon l'une quelconque des revendications précédentes, dans laquelle le monoacylglycéride comprend deux groupes vicinaux de liaison d'hydrogène.

5. Composition selon l'une quelconque des revendications précédentes, dans laquelle le monoacylglycéride est choisi dans le groupe constitué de monocaprylate de glycérol, monolaurate de glycérol, mono-isostéarate de glycérol, monostéarate de glycérol, mono-oléate de glycérol, et des combinaisons de ceux-ci.

6. Composition selon l'une quelconque des revendications précédentes, dans laquelle le monoacylglycéride est choisi dans le groupe constitué de monocaprylate de glycérol, monolaurate de glycérol, mono-isostéarate de glycérol, mono-oléate de glycérol, et des combinaisons de ceux-ci.

7. Composition selon l'une quelconque des revendications précédentes, dans laquelle la composition ne comprend pas plus de 0,1 partie en poids d'eau pour 1 partie en poids de digluconate de chlorhexidine.

8. Composition selon l'une quelconque des revendications précédentes, comprenant en outre un support.

9. Composition selon la revendication 8, dans laquelle le support comprend un polymère.

10. Composition selon la revendication 8 ou 9, dans laquelle le support comprend un solvant organique.

11. Composition selon l'une quelconque des revendications précédentes, comprenant en outre un agent thixotropique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6458341 B, Rozzi
- FR 2379284 A1


### Non-patent literature cited in the description


- **GRIFFIN WC.** *J. Soc. of Cosmetic Chemists,* 1954, vol. 5, 259
- **BELMARES, M. ; BLANCO, M. ; GODDARD, W.A. ; ROSS, R.B. ; CALDWELL, G ; CHOU, S.H. ; PHAM, J. ; OLOFSON, P.M. ; THOMAS, C.** *J. Comp. Chem.,* 2004, vol. 25 (15), 1814
- **RAPPE, A.K. ; GODDARD, W.A.** *J. Phys. Chem.,* 1991, vol. 95, 3358
- **MAYO, S.L. ; OLAFSON, B.D. ; GODDARD, W.A.** *J. Phys. Chem.,* 1990, vol. 94, 8897
- **ZHANG, H.P. ; XIONG, L ; LENG; Y ; FANG, L. ; QU, SHUXIN ; FENG, B. ; WENG, J. ; WANG, J.** *Acta Biomaterialia,* 2009, vol. 5 (4), 1169